(19) **Europäisches Patentamt / European Patent Office / Office européen des brevets**

(11) **EP 4 772 231 A1**

(12) **EUROPEAN PATENT APPLICATION**
published in accordance with Art. 153(4) EPC

(43) Date of publication:
**08.07.2026 Bulletin 2026/28**

(21) Application number: **23950630.6**

(22) Date of filing: **28.08.2023**

(51) International Patent Classification (IPC):
***A61M 25/09*** *(2006.01)*

(52) Cooperative Patent Classification (CPC):
**A61M 25/09**

(86) International application number:
**PCT/JP2023/030886**

(87) International publication number:
**WO 2025/046678 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Asahi Intecc Co., Ltd.
Seto-shi, Aichi 489-0071 (JP)**

(72) Inventor: **FUKUSHIMA Kensei
Seto-shi, Aichi 489-0071 (JP)**

(74) Representative: **Prinz & Partner mbB
Patent- und Rechtsanwälte
Rundfunkplatz 2
80335 München (DE)**

(54) **MEDICAL DEVICE SHAFT AND MEDICAL DEVICE**

(57) A medical device shaft includes a specific portion having a flattening of 2% or more and 6% or less, the flattening being defined as a value obtained by dividing a difference between an orthogonal diameter and a minimum diameter by the orthogonal diameter, where, in a cross-section orthogonal to an axial direction of the medical device shaft, a diameter having a smallest length is defined as the minimum diameter and a diameter in a direction orthogonal to a direction of the minimum diameter in the cross-section is defined as the orthogonal diameter.

FIG.1

**Description**

TECHNICAL FIELD

The technology disclosed in the present specification relates to a medical device shaft and a medical device.

BACKGROUND ART

For example, methods using catheters are widely used to treat or examine stenosis or occlusions (hereinafter referred to as "lesions") in blood vessels. A guidewire is used to guide the catheter to the lesion in the blood vessel. The guidewire includes a core shaft.

The guidewire is required to have rotational followability such that, when a proximal end portion held by a practitioner such as a physician is rotated, a distal end portion smoothly rotates in accordance therewith.

To improve the blood vessel selectivity of the guidewire, the practitioner performs a procedure called "shaping", in which the distal end portion of the guidewire is bent at a predetermined angle beforehand.

A known guidewire includes a portion having a flattening of 7% or more and 35% or less at the distal end portion of the core shaft in order to ensure good rotational followability while facilitating shaping of the distal end portion (see, e.g., Patent literature 1). The flattening of the core shaft is defined, in a cross-section orthogonal to the axial direction of the core shaft where the diameter having the smallest length is defined as a minimum diameter and the diameter in a direction orthogonal to the direction of the minimum diameter in the cross-section is defined as an orthogonal diameter, as a percentage obtained by dividing the difference between the orthogonal diameter and the minimum diameter by the orthogonal diameter.

CITATION LIST

Patent Literature

Patent Literature 1: WO2021/131019

SUMMARY OF INVENTION

TECHNICAL PROBLEM

The above-mentioned conventional technology has a problem in that it is difficult to prevent three-dimensional deformation when shaping the distal end portion of the guidewire while ensuring good rotational followability of the guidewire. It is preferable that the guidewire does not undergo three-dimensional deformation even when shaped. The expression that the guidewire does not undergo three-dimensional deformation is defined as meaning that the silhouette obtained by projecting the shaped guidewire onto a certain plane forms a shape approximating a straight line. This problem is not limited to the guidewire including the core shaft, but is a common problem for medical devices including medical device shafts.

The present specification discloses a technology that can solve the above-mentioned problems.

SOLUTION TO PROBLEM

The technology disclosed in the present specification can be achieved, for example, in the following aspects.

(1) A medical device shaft disclosed in the present specification includes a specific portion having a flattening of 2% or more and 6% or less, the flattening being defined as a value obtained by dividing a difference between an orthogonal diameter and a minimum diameter by the orthogonal diameter, where, in a cross-section orthogonal to an axial direction of the medical device shaft, a diameter having a smallest length is defined as the minimum diameter and a diameter in a direction orthogonal to a direction of the minimum diameter in the cross-section is defined as the orthogonal diameter.

The closer the cross-sectional shape of the medical device shaft is to a circular shape, the better the rotational followability of the medical device. When the cross-sectional shape of the medical device shaft is close to a circular shape, variation may occur in the shaping direction upon shaping, and the medical device may undergo three-dimensional deformation. The present medical device shaft includes a specific portion having a flattening of 2% or more and 6% or less. Although the cross-section of the specific portion is close to a circular shape, it is flattened to a certain extent, which can prevent variation in the shaping direction. Thus, the present medical device shaft can prevent three-dimensional deformation during shaping while ensuring good rotational followability.

(2) The medical device shaft may include a distal end side specific portion located on a distal end side of the specific portion, the distal end side specific portion having the flattening of 7% or more. According to the present configuration, variation in the shaping direction can be effectively prevented in the distal end side specific portion, and three-dimensional deformation during shaping can be effectively prevented.

(3) In the medical device shaft, the distal end side specific portion may include a curved portion that is curved about an axis parallel to the orthogonal diameter of the distal end side specific portion. According to the present configuration, variation in the shaping direction in the distal end side specific portion can be further effectively prevented, and three-dimensional deformation during shaping can be further effectively prevented.

(4) In the medical device shaft, the direction of the minimum diameter of the specific portion may be parallel to the direction of the minimum diameter of the distal end side specific portion. According to the present configuration, variation in the shaping direction in the specific portion and the distal end side specific portion can be effectively prevented, and three-dimensional deformation during shaping can be effectively prevented.

(5) In the medical device shaft, the flattening of the specific portion may be 4% or more and 6% or less. According to the present configuration, variation in the shaping direction can be effectively prevented, and three-dimensional deformation during shaping can be effectively prevented.

It should be noted that the technology disclosed in the present specification can be achieved in various aspects, for example, as a medical device shaft, as a medical device, as a method for producing the same, and the like.

BRIEF DESCRIPTION OF DRAWINGS

FIG. 1 is an explanatory diagram schematically illustrating a side view of a guidewire of a first embodiment.

FIG. 2 is a cross-sectional view of a core shaft taken along a line II-II in FIG. 1.

FIG. 3 is a cross-sectional view of the core shaft taken along a line III-III in FIG. 1.

FIG. 4 is an explanatory diagram illustrating an example of a guidewire shaping method.

FIG. 5 is an explanatory diagram schematically illustrating a side view of the guidewire of a second embodiment.

FIG. 6 is an explanatory diagram illustrating performance evaluation results for resistance to three-dimensional deformation upon repeated shaping.

FIG. 7 is an explanatory diagram illustrating the performance evaluation results for rotational followability.

EMBODIMENTS OF THE INVENTION

A. First embodiment:

A-1. Configuration of guidewire 100:

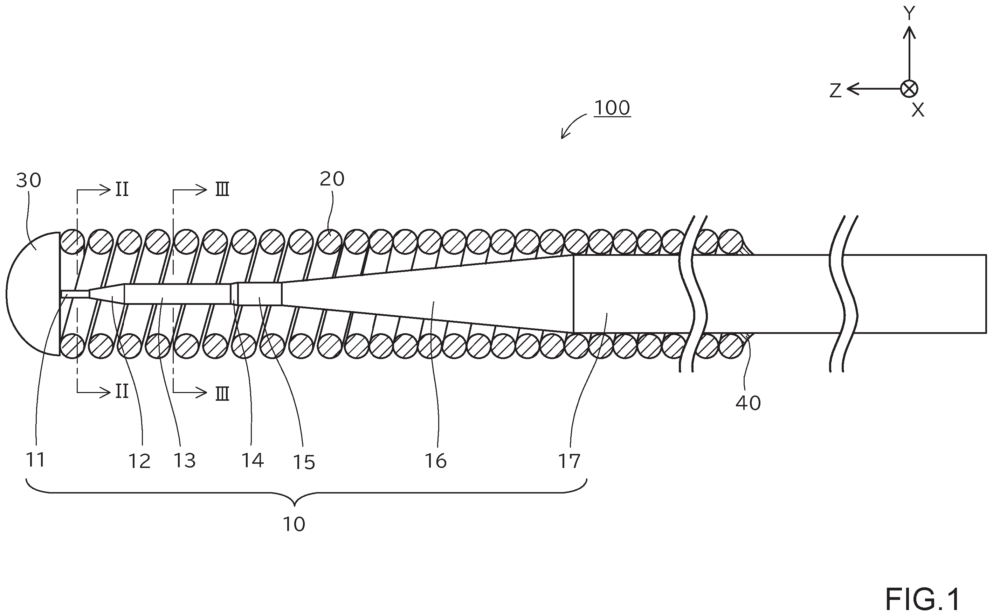

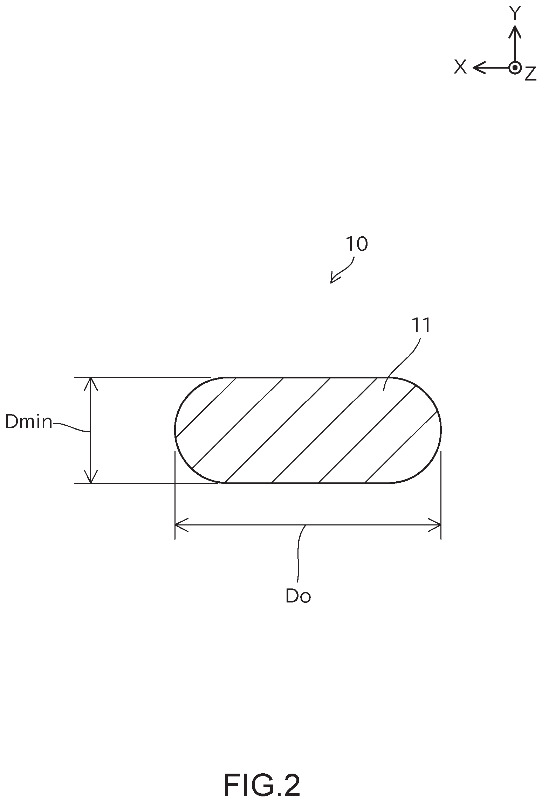

FIG. 1 is an explanatory diagram schematically illustrating a side view of a guidewire 100 of a first embodiment. In FIG. 1, mutually orthogonal X, Y, and Z axes are illustrated for specifying directions, and a side view of the guidewire 100 as viewed in the X-axis direction is illustrated. The positive Z-axis direction corresponds to the distal end side (tip end side) to be inserted into the body, and the negative Z-axis direction corresponds to the proximal end side (base end side) to be operated by a practitioner such as a physician. This also applies to FIG. 2 and subsequent drawings. FIG. 1 illustrates a cross section (specifically, a YZ cross section) of a coil body 20 described below. In FIG. 1, the guidewire 100 is illustrated in a state in which the entire guidewire 100 is linearly oriented substantially parallel to the Z-axis direction. However, the guidewire 100 has flexibility sufficient to allow it to be bent. In the present specification, with respect to the guidewire 100 and each constituent member thereof, the end on the distal end side is referred to as the "distal end", the distal end and its vicinity are referred to as the "distal end portion", the end on the proximal end side is referred to as the "proximal end," and the proximal end and its vicinity are referred to as the "proximal end portion".

The guidewire 100 is a medical device. For example, the guidewire 100 is inserted into a blood vessel to guide another medical device (not illustrated), such as a catheter, to a lesion in the blood vessel. The guidewire 100 includes a core shaft 10. The guidewire 100 includes the coil body 20. The guidewire 100 includes a distal end side joint portion 30. The guidewire 100 includes a proximal end side joint portion 40.

The core shaft 10 is an elongated member. The core shaft 10 includes a first portion 11, a second portion 12, a third portion 13, a fourth portion 14, a fifth portion 15, a sixth portion 16, and a seventh portion 17. The first portion 11, the second portion 12, the third portion 13, the fourth portion 14, the fifth portion 15, the sixth portion 16, and the seventh portion 17 are arranged in order from the distal end side toward the proximal end side. The core shaft 10 is an example of the medical device shaft.

In the present embodiment, the first portion 11, the third portion 13, the fifth portion 15, and the seventh portion 17 have a constant cross-sectional shape at each position along the axial direction. The cross-sectional area of the third portion 13 is larger than that of the first portion 11. The cross-sectional area of the fifth portion 15 is larger than that of the third portion 13. The cross-sectional area of the seventh portion 17 is larger than that of the fifth portion 15. The second portion 12, the fourth portion 14, and the sixth portion 16 are smoothly connected, along the axial direction, to the cross-sectional shapes of other adjacent portions. The second portion 12, the fourth portion 14, and the sixth portion 16 are tapered portions whose cross-sectional areas gradually increase from the distal end side to the proximal end side.

Examples of a material for forming the core shaft 10 include a metal material, more specifically, stainless steel (SUS302, SUS304, SUS316, etc.), a Ni-Ti alloy, a piano wire, a nickel-chromium alloy, a cobalt alloy, and tungsten. The configuration of the core shaft 10 is described in detail below.

The coil body 20 is a hollow cylindrical member formed by spirally winding a wire. The coil body 20 is disposed so as to surround the outer periphery of a distal end portion of the core shaft 10.

Examples of a material for forming the coil body 20 include a metal material, more specifically, a radiolucent material such as stainless steel (SUS302, SUS304, SUS316, etc.), a Ni-Ti alloy, a piano wire, a nickel-chromium alloy, or a cobalt alloy, and a radiopaque material such as gold, platinum, tungsten, or an alloy containing these elements (e.g., a platinum-nickel alloy).

The distal end side joint portion 30 joins the distal end portion of the core shaft 10 to a distal end portion of the coil body 20. The outer peripheral surface of the distal end side joint portion 30 on the distal end side is formed as a smooth surface (e.g., a substantially hemispherical surface). The proximal end side joint portion 40 joins the core shaft 10 and the proximal end portions of the coil body 20. Examples of a material for forming the distal end side joint portion 30 and the proximal end side joint portion 40 include metal solder such as silver solder, gold solder, zinc, a Sn-Ag alloy, or an Au-Sn alloy, and an adhesive such as an epoxy adhesive.

A-2. Detailed configuration of core shaft 10:

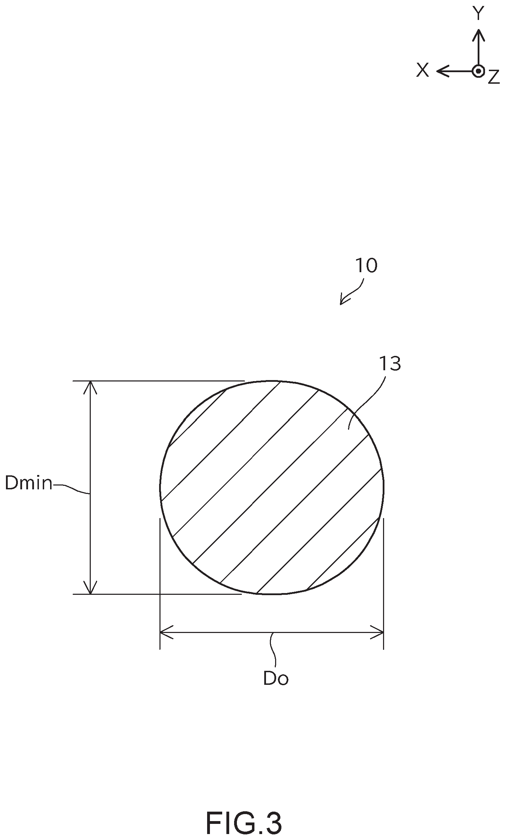

FIG. 2 is a diagram illustrating a cross section of the core shaft 10 taken along a line II-II in FIG. 1. FIG. 3 is a diagram illustrating a cross section of the core shaft 10 taken along a line III-III in FIG. 1. In the present specification, in a cross section (XY cross section) orthogonal to the axial direction (Z-axis direction) of each portion of the core shaft 10, the diameter with the smallest length is defined as a minimum diameter $D_{min}$, and the diameter in the direction orthogonal to the direction of the minimum diameter $D_{min}$ is defined as an orthogonal diameter $D_o$. The flattening F of each portion of the core shaft 10 is defined as a percentage obtained by dividing the difference between the orthogonal diameter $D_o$ and the minimum diameter $D_{min}$ by the orthogonal diameter $D_o$. That is, the flattening F of each portion of the core shaft 10 is defined by the following formula (1).

$$F=((D_o-D_{min})/D_o)\times 100 \cdots (1)$$

As illustrated in FIG. 2, the cross section of the first portion 11 of the core shaft 10 has a relatively flat shape. The cross section of the first portion 11 is, for example, substantially elliptical. The flattening F of the first portion 11 is 7% or more. The flattening F of the first portion 11 may be 10% or more and 70% or less, 20% or more and 60% or less, or 30% or more and 50% or less. The direction of the minimum diameter $D_{min}$ of the first portion 11 is the Y-axis direction, and the direction of the orthogonal diameter $D_o$ of the first portion 11 is the X-axis direction. The length of the first portion 11 may be, for example, about 1 mm to 10 mm. The first portion 11 of the core shaft 10 is an example of the distal end side specific portion.

As illustrated in FIG. 3, the cross section of the third portion 13 of the core shaft 10 has a shape that is relatively close to a circular shape. The flattening F of the third portion 13 is 2% or more and 6% or less. The flattening F of the third portion 13 may be 4% or more. The direction of the minimum diameter $D_{min}$ of the third portion 13 is the Y-axis direction, and the direction of the orthogonal diameter $D_o$ of the third portion 13 is the X-axis direction. That is, the direction of the minimum diameter $D_{min}$ of the third portion 13 and the direction of the minimum diameter $D_{min}$ of the first portion 11 are parallel to each other, and the direction of the orthogonal diameter $D_o$ of the third portion 13 and the direction of the orthogonal diameter $D_o$ of the first portion 11 are parallel to each other. The distal end of the third portion 13 may be located, for example, at a position about 5 mm to 10 mm from the distal end of the core shaft 10, and the length of the third portion 13 may be, for example, about 3 mm to 15 mm. The third portion 13 of the core shaft 10 is an example of the specific portion.

The flattening F of the second portion 12 of the core shaft 10 is close to the flattening F of the first portion 11, and is, for example, 7% or more. The flattening F of the second portion 12 may be 10% or more, 20% or more, or 30% or more, and may be 70% or less, 60% or less, or 50% or less. The cross sections of the fourth portion 14 of the core shaft 10 and the portions on the proximal end side thereof are substantially circular. The flattening F of these portions is, for example, less than 2%.

The core shaft 10 can be produced, for example, by preparing a member having a circular cross section at each portion and then performing flattening processing such as press processing according to the flattening set at each portion.

A-3. Guidewire 100 shaping method:

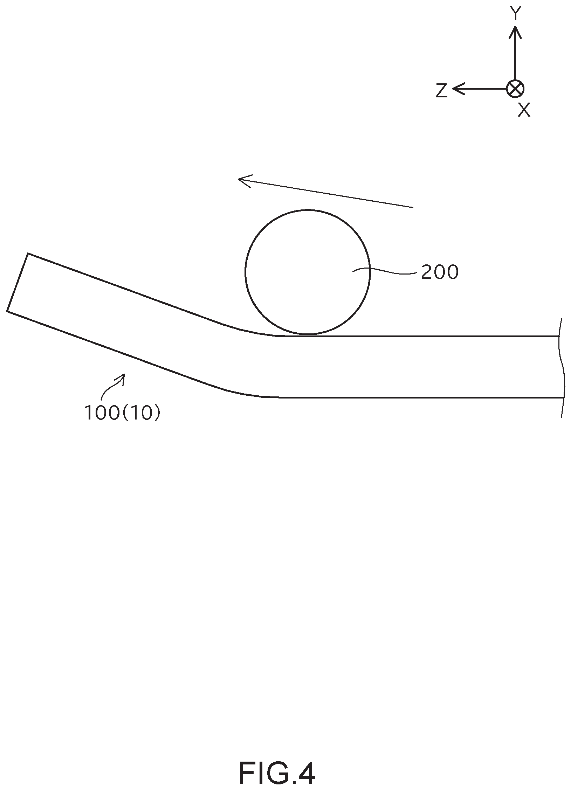

FIG. 4 is an explanatory diagram illustrating an example of a method for shaping the guidewire 100. FIG. 4 illustrates a method for performing a procedure called "shaping", in which the distal end portion of the guidewire 100 is bent at a predetermined angle to improve blood vessel selectivity when the practitioner uses the guidewire 100.

As illustrated in FIG. 4, the practitioner moves a shaping needle 200 along the distal end portion of the guidewire 100 and performs shaping in one direction. At this time, the practitioner bends the guidewire 100 around an axis (X-axis) parallel to the orthogonal diameter $D_o$ of the first portion 11 and the third portion 13 of the core shaft 10. In other words, the practitioner bends the guidewire 100 along the direction of the minimum diameter $D_{min}$ (Y-axis direction) of the first portion 11 and the third portion 13 of the core shaft 10. Thereafter, the practitioner inserts the guidewire 100 into a blood vessel for use. The practitioner, for example, during vessel selection, rotates the proximal end portion of the guidewire 100 about the Z-axis to thereby rotate the distal end portion of the guidewire 100.

A-4. Effects of present embodiment:

As described above, the core shaft 10 for the guidewire 100 of the present embodiment include the third portion 13 having a flattening F of 2% or more and 6% or less.

The rotational followability of the guidewire 100 improves as the cross-sectional shape of the core shaft 10 approaches a circular shape. If the cross-sectional shape of the core shaft 10 approaches a circular shape, variations in the shaping direction may occur, and the guidewire 100 may undergo three-dimensional deformation. The flattening F of the third portion 13 of the core shaft 10 of the present embodiment is 2% or more and 6% or less. Although the cross-sectional shape of the third portion 13 is close to a circular shape, it is flattened to a certain extent, which can prevent variations in the shaping direction. Thus, the guidewire 100 of the present embodiment can prevent three-dimensional deformation due to shaping while ensuring good rotational followability.

The flattening F of the third portion 13 of the core shaft 10 may be 4% or more and 6% or less. Such a configuration can effectively prevent variations in the shaping direction and effectively prevent three-dimensional deformation due to shaping.

The core shaft 10 for the guidewire 100 of the present embodiment include the first portion 11 that is located on the distal end side of the third portion 13 and has a flattening F of 7% or more. Thus, the core shaft 10 of the present embodiment can effectively prevent variations in the shaping direction in the first portion 11, and can effectively prevent three-dimensional deformation due to shaping.

In the core shaft 10 for the guidewire 100 of the present embodiment, the direction of the minimum diameter $D_{min}$ of the third portion 13 and the direction of the minimum diameter $D_{min}$ of the first portion 11 are parallel to each other. Thus, according to the core shaft 10 of the present embodiment, it is possible to effectively prevent variations in the shaping directions of the first portion 11 and the third portion 13, and it is possible to effectively prevent three-dimensional deformation during shaping.

B. Second embodiment:

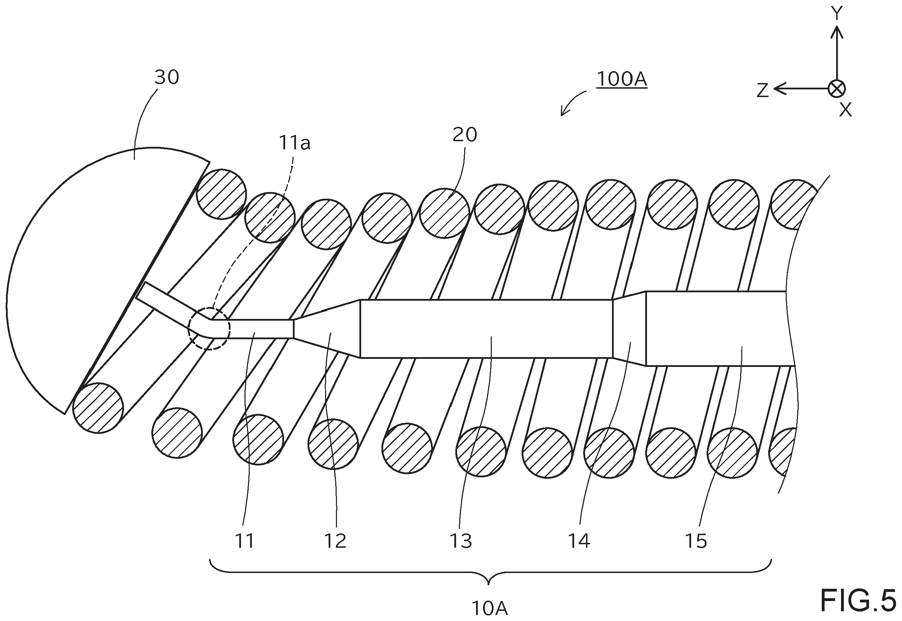

FIG. 5 is an explanatory diagram schematically illustrating a side view of a guidewire 100A of a second embodiment. In the following description, with respect to the configuration of the guidewire 100A of the second embodiment, components that are the same as those of the guidewire 100 of the first embodiment described above are denoted by the same reference numerals, and description thereof is omitted as appropriate.

In a core shaft 10A of a guidewire 100A of the second embodiment, the first portion 11 includes a curved portion 11a. The curved portion 11a is a portion that is pre-bent around an axis (X-axis) parallel to the orthogonal diameter $D_o$ of the first portion 11 (see FIG. 2). Due to the presence of the curved portion 11a in the core shaft 10A, the guidewire 100A, in which the coil body 20 (FIG. 1) is joined to the core shaft 10A, is also curved at the position of the curved portion 11a. A bending angle of the curved portion 11a may be, for example, 10 degrees or more and 70 degrees or less, 20 degrees or more and 60 degrees or less, or 30 degrees or more and 50 degrees or less. A position of the curved portion 11a may be, for example, about 0.5 mm to 5 mm from the distal end of the core shaft 10A.

The guidewire 100A of the second embodiment can be produced by joining the coil body 20 to the core shaft 10A, and then performing bending to form the curved portion 11a.

In the core shaft 10A for the guidewire 100A of the second embodiment, the first portion 11 includes the curved portion 11a, which makes it possible to more effectively prevent variations in the shaping direction in the first portion 11, and more effectively prevent three-dimensional deformation due to shaping.

C. Performance evaluation:

Performance evaluation was conducted on the core shaft for a guidewire with respect to resistance to three-dimensional deformation upon repeated shaping and rotational followability.

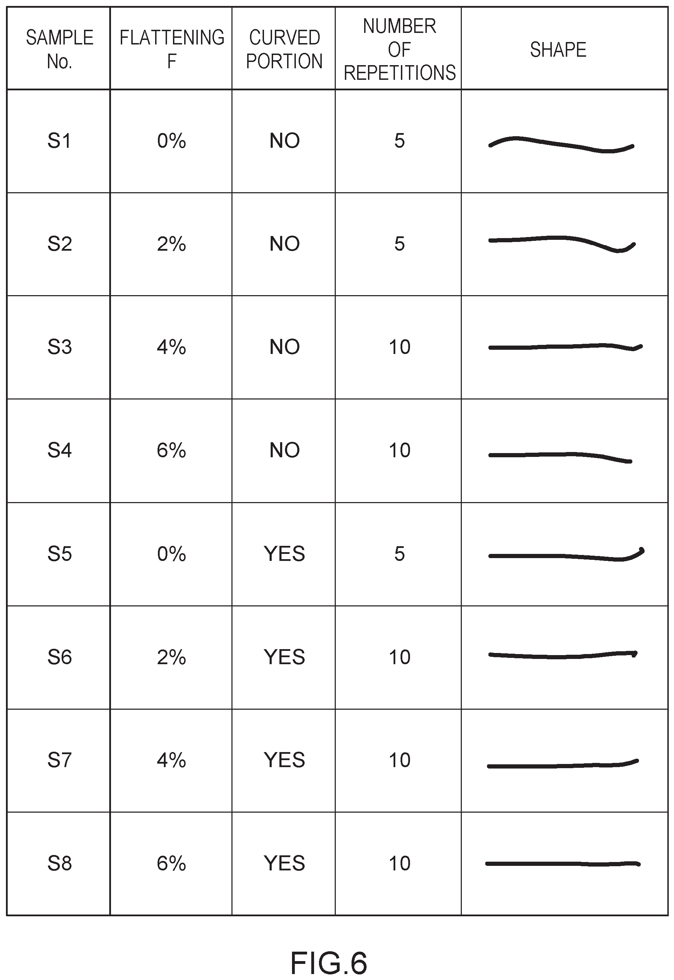

FIG. 6 is an explanatory diagram illustrating performance evaluation results for the resistance to three-dimensional deformation upon repeated shaping. In actual surgery, shaping is usually performed only once. In the performance evaluation results, shaping and reshaping were repeated to evaluate three-dimensional deformation. As illustrated in FIG. 6, in the performance evaluation results, multiple core shafts 10 having different flattening F of the distal end portions (the third portions 13 in the above-described embodiments) were prepared. Samples of the guidewires 100 including the core shafts 10 were produced. Samples S1 to S4 include the core shafts 10 without the curved portions 11a (corresponding to the first embodiment described above). Samples S5 to S8 include the core shafts 10 with the curved portions 11a (corresponding to the second embodiment described above). For each sample, the guidewire 100 was shaped from an initial, straight state to a height of 5 mm, and then reshaped to a height of 1 mm or less. This process was repeated the number of times illustrated in FIG. 6. The shape of the guidewires 100 after the repeated processing is illustrated in FIG. 6. The shape illustrated in FIG. 6 represents the silhouette obtained by projecting the guidewires 100 onto a ZX plane. This silhouette is a straight line before shaping. After shaping and reshaping, if no three-dimensional deformation occurs, the silhouette remains as a straight line.

As illustrated in FIG. 6, for both samples without the curved portions 11a (S1-S4) and samples with the curved portions 11a (S5-S8), the ones with a flattening F of 2% or more and 6% or less (S2 to S4, S6 to S8) showed relatively good prevention of three-dimensional deformation upon repeated shaping. In particular, the ones with a flattening F of 4% or more and 6% or less (S3 and S4, S7 and S8) showed even better prevention of three-dimensional deformation upon repeated shaping. When comparing the samples with the same flattening F, the samples with the curved portions 11a (S6 to S8) showed better prevention of three-dimensional deformation upon repeated shaping than the samples without the curved portions 11a (S2 to S4).

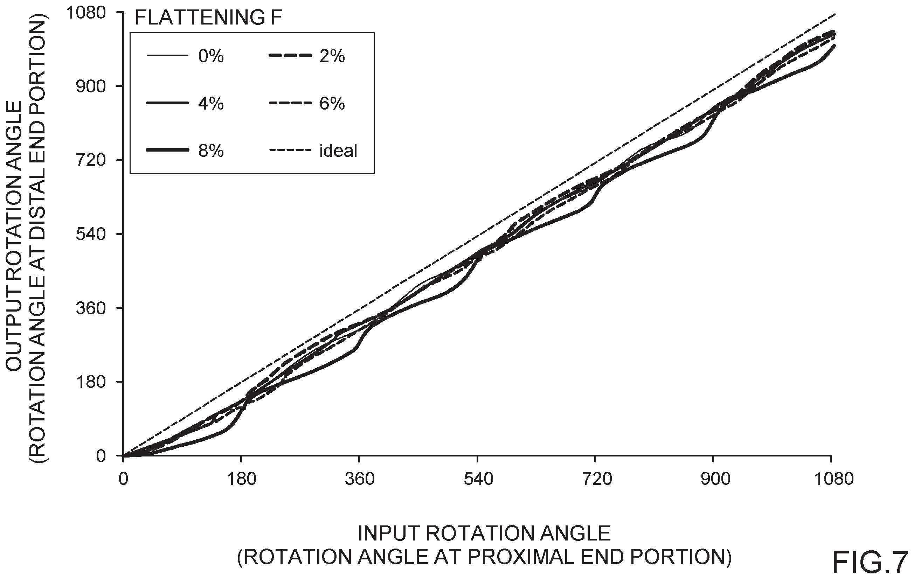

FIG. 7 is an explanatory diagram illustrating the performance evaluation results of rotational followability. As illustrated in FIG. 7, this performance evaluation evaluated the relationship between the angle at which the proximal end portion of the guidewire 100 was rotated (input rotation angle) and the corresponding angle at which the distal end portion of the guidewire 100 was rotated (output rotation angle). It can be said that the closer the relationship lies to the line in which the input rotation angle and the output rotation angle coincide ("ideal" line in FIG. 7), the higher the rotational followability. This performance evaluation was conducted using the samples with the curved portions 11a.

As illustrated in FIG. 7, the larger the flattening F of the distal end portion of the core shaft 10, the lower the rotational followability. In the sample in which the flattening F of the distal end portion of the core shaft 10 was 8%, the input rotational torque accumulated as strain, and then a whip occurred in which the distal end portion suddenly rotated all at once. Since the presence or absence of the curved portion 11a is thought to have almost no effect on the rotational followability, it is thought that the same result would be obtained with the core shafts 10 that do not have the curved portions 11a.

In view of the above performance evaluation results, if the core shaft 10 for the guidewire 100 includes the third portion 13 having a flattening F of 2% or more and 6% or less, it is possible to prevent three-dimensional deformation during shaping while ensuring good rotational followability. It can be said that if the flattening F of the third portion 13 of the core shaft 10 is 4% or more and 6% or less, three-dimensional deformation during shaping can be effectively prevented. It can be said that if the first portion 11 of the core shaft 10 includes the curved portion 11a, three-dimensional deformation due to shaping can be prevented even more effectively.

D. Modifications:

The technology disclosed in the present specification is not limited to the above-described embodiments, and can be modified in various forms without departing from the gist thereof. For example, the following modifications are possible.

The configuration of the guidewire 100 in the above-described embodiments is merely an example and can be modified in various ways. For example, the core shaft 10 does not need to include a portion having a flattening of 7% or more on the distal end side of the third portion 13.

The core shaft 10 may not include at least one of the first portion 11 to the seventh portion 17, as long as it includes the third portion 13 having a flattening of 2% or more and 6% or less.

The direction of the minimum diameter $D_{min}$ of the third portion 13 of the core shaft 10 and the direction of the minimum diameter $D_{min}$ of the first portion 11 need not be parallel to each other.

The technology disclosed in the present specification is not limited to the core shaft 10 for the guidewire 100, but can be generally applicable to medical device shafts.

DESCRIPTION OF REFERENCE NUMERALS

10: Core shaft

11: First portion

11a: Curved portion

12: Second portion

13: Third portion

14: Fourth portion

15: Fifth portion

16: Sixth portion

17: Seventh portion

20: Coil body

30: Tip end side joint portion

40: Base end side joint portion

100: Guidewire

200: Shaping needle

$D_{min}$: Minimum diameter

$D_o$: Orthogonal diameter

## Claims

1. A medical device shaft comprising a specific portion having a flattening of 2% or more and 6% or less, the flattening being defined as a value obtained by dividing a difference between an orthogonal diameter and a minimum diameter by the orthogonal diameter, where, in a cross-section orthogonal to an axial direction of the medical device shaft, a diameter having a smallest length is defined as the minimum diameter and a diameter in a direction orthogonal to a direction of the minimum diameter in the cross-section is defined as the orthogonal diameter.

2. The medical device shaft according to claim 1, further comprising a distal end side specific portion located on a distal end side of the specific portion, the distal end side specific portion having the flattening of 7% or more.

3. The medical device shaft according to claim 2, wherein
the distal end side specific portion includes a curved portion that is curved about an axis parallel to the orthogonal diameter of the distal end side specific portion.

4. The medical device shaft according to claim 2 or 3, wherein
the direction of the minimum diameter of the specific portion is parallel to the direction of the minimum diameter of the distal end side specific portion.

5. The medical device shaft according to any one of claims 1 to 4, wherein
the flattening of the specific portion is 4% or more and 6% or less.

6. A medical device comprising the medical device shaft according to any one of claims 1 to 5.

EP 4 772 231 A1

FIG.1

FIG.2

Y

X ← ⊙ Z

10

13

Dmin

Do

FIG.3

FIG.4

FIG.5

| SAMPLE No. | FLATTENING F | CURVED PORTION | NUMBER OF REPETITIONS | SHAPE |
|---|---|---|---|---|
| S1 | 0% | NO | 5 | |
| S2 | 2% | NO | 5 | |
| S3 | 4% | NO | 10 | |
| S4 | 6% | NO | 10 | |
| S5 | 0% | YES | 5 | |
| S6 | 2% | YES | 10 | |
| S7 | 4% | YES | 10 | |
| S8 | 6% | YES | 10 | |

FIG.6

FIG.7

## INTERNATIONAL SEARCH REPORT

| International application No. |
|---|
| **PCT/JP2023/030886** |

### A. CLASSIFICATION OF SUBJECT MATTER

*A61M 25/09*(2006.01)i
FI: A61M25/09 510

According to International Patent Classification (IPC) or to both national classification and IPC

### B. FIELDS SEARCHED

Minimum documentation searched (classification system followed by classification symbols)

A61M25/09

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2005-103171 A (ASAHI INTECC CO., LTD.) 21 April 2005 (2005-04-21) paragraph [0030], fig. 3 | 1-2, 4-6 |
| Y | | 3 |
| Y | JP 2013-183984 A (TERUMO KABUSHIKI KAISHA) 19 September 2013 (2013-09-19) paragraph [0031], fig. 1 | 3 |
| Y | JP 2016-158933 A (ASAHI INTECC CO., LTD.) 05 September 2016 (2016-09-05) paragraph [0038], fig. 7 | 3 |
| A | WO 2021/131019 A1 (ASAHI INTECC CO., LTD.) 01 July 2021 (2021-07-01) entire text, all drawings | 1-6 |
| A | WO 2014/162392 A1 (TERUMO KABUSHIKI KAISHA) 09 October 2014 (2014-10-09) entire text, all drawings | 1-6 |

☐ Further documents are listed in the continuation of Box C.    ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 October 2023** | **07 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/030886**

| Patent document cited in search report | | | Publication date (day/month/year) | Patent family member(s) | | | Publication date (day/month/year) |
|---|---|---|---|---|---|---|---|
| JP | 2005-103171 | A | 21 April 2005 | US 2005/0096568 A1 paragraph [0054], fig. 6-7 EP 1520600 A1 CN 1618478 A | | | |
| JP | 2013-183984 | A | 19 September 2013 | (Family: none) | | | |
| JP | 2016-158933 | A | 05 September 2016 | (Family: none) | | | |
| WO | 2021/131019 | A1 | 01 July 2021 | US 2022/0296860 A1 entire text, all drawings | | | |
| WO | 2014/162392 | A1 | 09 October 2014 | US 2016/0008586 A1 entire text, all drawings | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021131019 A